# EUROPEAN PATENT SPECIFICATION

(11) **EP 3 045 527 B1**
(45) Date of publication and mention of the grant of the patent: **11.08.2021**
(21) Application number: 14843961.5
(22) Date of filing: 10.09.2014
(51) Int. Cl.: C12M 3/00

(54) **CELL CULTURE VESSEL**
ZELLKULTURGEFÄSS
RÉCIPIENT DE CULTURE CELLULAIRE

(30) Priority: 11.09.2013 JP 2013188598
(43) Date of publication of application: 20.07.2016
(73) Proprietor: Dai Nippon Printing Co., Ltd., Tokyo 162-8001 (JP)
(72) Inventor: BABA Takuma, Tokyo 162-8001 (JP); KAGOTA Masanori, Tokyo 162-8001 (JP); AKAI Tomonori, Tokyo 162-8001 (JP); OKUMURA Koichiro, Tokyo 162-8001 (JP); KIMURA Atsumi, Tokyo 162-8001 (JP)
(74) Representative: Beck Greener LLP
(86) International application number: PCT/JP2014/073860
(87) International publication number: WO 2015/037595

(56) References cited:
- WO-A1-2013/042360
- CA-A1- 2 848 875
- JP-A- 2009 017 810
- JP-A- 2009 242 583
- JP-A- 2010 200 748
- JP-A- 2013 138 629
- US-A1- 2010 170 790
- US-A1- 2010 221 768
- DATABASE WPI Week 201348 Thomson Scientific, London, GB; AN 2013-L80293 XP002768305, -& JP 2013 138629 A (DAINIPPON PRINTING CO LTD) 18 July 2013 (2013-07-18)

## Description

### TECHNICAL FIELD

The present invention relates to a cell culture vessel for culturing cells such as fertilized eggs requiring individual management. The cell culture vessel comprises a bottom and a side wall, wherein at least one microwell for containing a cell is disposed on the bottom, the microwell has a bottom surface, a side surface, and an opening, and a convex-concave structure is formed on the side surface of the microwell, wherein the height of the linear convex part with respect to the concave part is 5 µm or more.

### BACKGROUND ART

Sperms and eggs are fertilized *in vitro* in a culture system to prepare fertilized eggs (zygotes) and the fertilized eggs can be further cultured until they are developed into cleavage, morula and blastocyst stages and hatched from zona pellucida into the hatched blastocyst stage. A technique of implanting a fertilized egg in the cleavage to blastocyst stage into the uterus to obtain an offspring, i.e., assisted reproductive technology (ART) has been established not only in the field of livestock but also in medical treatment for human infertility.

However, the rate of successful pregnancy by *in vitro* fertilization is not always high. For example, in human, the rate of successful pregnancy still remains at about 25 to 35%. As a cause of this, it is pointed out that the probability of obtaining a good fertilized egg suitable for implantation to the uterus by culture is not high. The fertilized eggs obtained by culture are individually subjected to microscopic observation by an expert to determine whether the fertilized eggs of good quality are suitable for uterus implantation.

In the *in vitro* fertilization, a micro-drop method, in which a drop of culture medium is prepared in a vessel, and a fertilized egg is introduced in the drop and subjected to *in vitro* culture, is frequently used. In the micro-drop method known in the art, a petri dish of 30 to 60 mm in diameter having a uniform flat bottom surface is used as a cell culture vessel. On the bottom surface of the petri dish, a plurality of drops of culture medium are prepared at intervals and cells are cultured in the drops. Such a cell culture method has been used.

When a drop is prepared in a petri dish conventionally used, the position of a fertilized egg changes depending upon the cellular motion of the fertilized egg itself and convection within the drop. Thus, it is difficult to identify the fertilized egg cultured in the drop and monitored. Because of the problem, it has been desired to develop means for controlling the position of a fertilized egg.

To more efficiently obtain the culture effect of a fertilized egg, it is preferable to use interaction of fertilized eggs to each other (paracrine effect). For controlling the position of a fertilized egg while using the effect, a system in which microwells having the same size as that of the fertilized egg are formed in the bottom surface of a petri dish and drop of culture medium is added so as to cover the microwells, and then fertilized eggs are disposed in microwells filled with the culture medium and cultured is known. Owing to the system, a plurality of fertilized eggs can be cultured in a small amount of culture medium while successfully controlling the positions of a plurality of fertilized eggs so as to enable monitoring of individual eggs, and the paracrine effect can be used.

### CITATION LIST

### Patent Literature

Patent Literature 1: JP Patent No. 4724854

### SUMMARY OF INVENTION

### Technical Problem

The present inventors have found that when a cell culture vessel with a microwell is used for culturing a fertilized egg, bubbles remain in the microwell at the time of adding a culture medium due to a small size of the microwell and consequently operation for removing the bubbles is required, making the procedure cumbersome, hence problematic.

For this reason, the present invention has an object to provide a cell culture vessel capable of making less likely to leave bubbles and improving culture operability for the cell culture conducted by the microdrop technique using a culture vessel with a microwell for containing a cell.

### Solution to Problem

The present inventors have found that the above problem can be solved when a convex-concave structure is formed on the side surface of the microwell.

More specifically, the present invention encompasses the following inventions.
(1) A cell culture vessel comprising a bottom and a side wall,
   wherein
   at least one microwell for containing a cell is disposed on the bottom,
   the microwell has a bottom surface, a side surface, and an opening, and
   a convex-concave structure is formed on the side surface of the microwell,
   wherein the height of the linear convex part with respect to the concave part is 5 µm or more.
(2) The cell culture vessel according to (1), wherein the side surface of the microwell is a hydrophilic surface.
(3) The cell culture vessel according to (1) or (2), wherein the convex-concave structure is configured by at least two linear convex parts and concave parts.
(4) The cell culture vessel according to (1) or (2), wherein the convex-concave structure is configured by at least two dot projections and/or at least two dot recessions.
(5) The cell culture vessel according to any one of (1) to (4), wherein an opening width of the opening of the microwell is 0.1 mm or more and 1 mm or less.
(6) The cell culture vessel according to any one of (1) to (5), wherein the bottom surface of the microwell is a smooth surface.
(7) The cell culture vessel according to (6), wherein a surface roughness of the bottom surface of the microwell has a maximum height (Ry) of 3 µm or less.
(8) The cell culture vessel according to any one of (3) and (5) to (7), wherein, in the convex-concave structure configured by at least two linear convex parts and concave parts, a width of the convex part and a width of the concave part range from 1/320 to 1/4 of a length of an outer peripheral portion of the opening.
(9) The cell culture vessel according to any one of (3) and (5) to (8), wherein, in the convex-concave structure configured by at least two linear convex parts and concave parts, a height of the convex part with respect to the concave part ranges from 1/1000 to 1/1.5 of the opening width of the opening.
(10) The cell culture vessel according to any one of (3) and (5) to (9), wherein, in the convex-concave structure configured by at least two linear convex parts and concave parts, a ratio of the height of the convex part to the width of the convex part ranges from 0.1 to 1.5.
(11) The cell culture vessel according to any one of (3) and (5) to (10), wherein, in the convex-concave structure configured by at least two linear convex parts and concave parts, a width of the convex part and a width of the concave part range from 15 to 70 µm.
(12) The cell culture vessel according to any one of (3) and (5) to (11), wherein, in the convex-concave structure configured by at least two linear convex parts and concave parts, a height of the convex part with respect to the concave part ranges from 1 to 70 µm.
(13) The cell culture vessel according to any one of (3) and (5) to (12),
   wherein
   the bottom surface of the microwell has an inverted conical shape having a downward inclined plane to the surface of the bottom of the cell culture vessel,
   the side surface of the microwell is inclined against a straight line orthogonal to the surface of the bottom of the cell culture vessel, and
   in the convex-concave structure configured by at least two linear convex parts and concave parts, a cross-section of the convex part has a square shape or an arc shape on a plane passing the centroid of the bottom surface of the microwell and orthogonal to a plane parallel with the bottom of the cell culture vessel.
(14) A cell culture vessel comprising a bottom and a side wall, according to (1) to (13),
   wherein
   at least one microwell for containing a cell is disposed on the bottom,
   the microwell has a bottom surface, a side surface, and an opening, and
   the bottom of the cell culture vessel, including the microwell and a circumference of the microwell, has a hydrophilic surface.
(15) The cell culture vessel according to any one of (2) to (14), wherein a water contact angle of the hydrophilic surface is 70° or less.

### Advantageous Effects of Invention

According to the present invention, a cell culture vessel with improved culture operability can be provided.

### BRIEF DESCRIPTION OF DRAWING

Figure 1 is a schematic view showing the top view of an embodiment of the cell culture vessel according to the present invention.
Figure 2 is a schematic view showing a vertical cross-sectional view of an embodiment of the cell culture vessel according to the present invention.
Figure 3 is a schematic view showing an enlarged perspective view of an embodiment of the microwell according to the present invention.
Figure 4 is a schematic view of the top view of the microwell shown in Figure 3.
Figure 5 is a schematic view showing an enlarged perspective view of an embodiment of the microwell according to the present invention.
Figure 6 is a schematic view showing an enlarged perspective view of an embodiment of the microwell according to the present invention.
Figure 7 is a schematic view showing a vertical cross-sectional view of an embodiment of the cell culture method using the cell culture vessel according to the present invention.
Figure 8A is a schematic view showing an enlarged perspective view of an embodiment of the microwell according to the present invention.
Figure 8B is a schematic view showing the top view of the microwell shown in Figure 8A.
Figure 8C is a schematic view showing a cross-sectional view taken along line X-X' of the microwell shown in Figure 8B.
Figure 9A is a schematic view showing an enlarged perspective view of an embodiment of the microwell according to the present invention.
Figure 9B is a schematic view showing the top view of the microwell shown in Figure 9A.
Figure 9C is a schematic view showing a cross-sectional view taken along line X-X' of the microwell shown in Figure 9B.
Figure 10A is a schematic view showing an enlarged perspective view of an embodiment of the microwell according to the present invention.
Figure 10B is a schematic view showing the top view of the microwell shown in Figure 10A.
Figure 10C is a schematic view showing a cross-sectional view taken along line X-X' of the microwell shown in Figure 10B.

### DESCRIPTION OF EMBODIMENTS

Hereinbelow, the present invention is described.

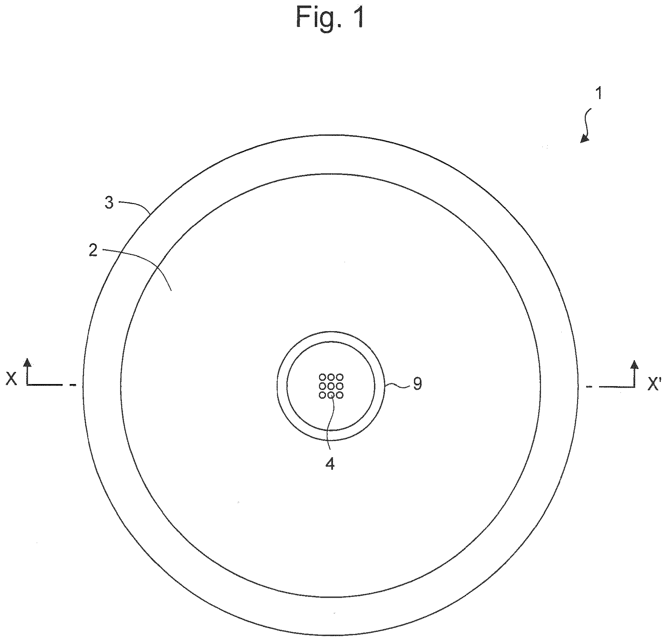

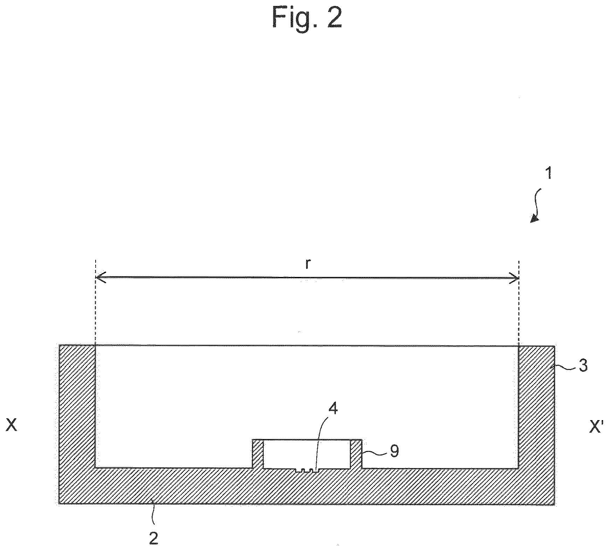

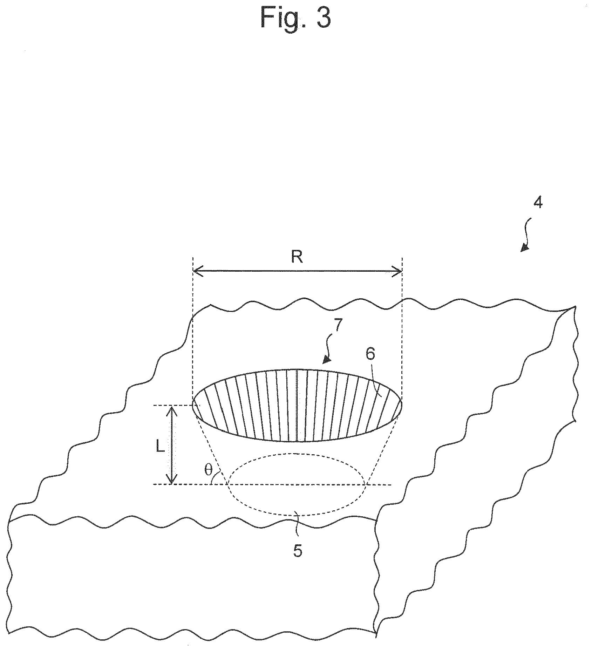

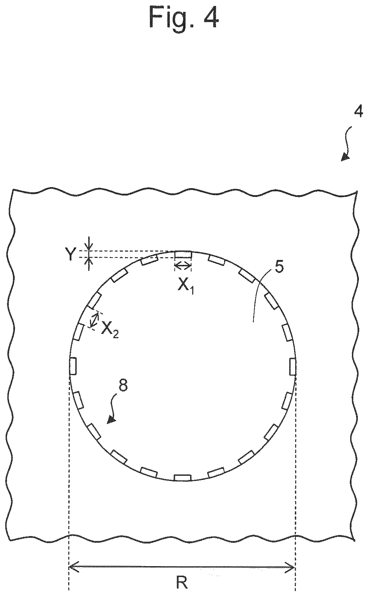

Figures 1 to 4 show schematic views of embodiments of the cell culture vessel according to the present invention. Figure 1 shows a top view, Figure 2 shows a vertical cross-sectional view, Figure 3 shows an enlarged perspective view of the microwell, and Figure 4 shows an enlarged top view of the microwell.

As shown in Figures 1 to 4, a cell culture vessel 1 of the present invention has
a bottom 2 and a side wall 3,
   wherein
at least one microwell 4 for containing a cell is disposed on the bottom 2,
the microwell 4 has a bottom surface 5, a side surface 6, and an opening 7, and
a convex-concave structure 8 is formed on the side surface 6 of the microwell.

Note that, in Figure 1 and Figure 2, an inner wall 9 surrounding a plurality of microwells is shown but the inner wall is not an essential component.

When the convex-concave structure is formed on the side surface of the microwell for containing a cell, bubbles are less likely to remain at the time of adding dropwise a culture medium to the microwell, obviating an operation for removing bubbles, whereby the culture operability can be improved.

The microwell has the bottom surface, the side surface, and the opening, and configures a recessed part. The convex-concave structure formed on the side surface of the microwell is not particularly limited, but the structure is preferably configured by fine convex parts and concave parts as formed on the side surface of the fine microwell. Additionally, for enhancing the removal of bubbles throughout the entire side surface of the microwell, the convex-concave structure is preferably formed throughout the entire side surface of the microwell. In the present invention, the expression "the convex-concave structure is formed throughout the entire side surface of the microwell" means that the convex-concave structure is formed on, for example, 80% or more, preferably 90% or more, more preferably 95% or more, particularly preferably 98% or more, and especially preferably about 100% of a region with respect to the total area of the side surface of the microwell. The convex-concave structure may also be formed, in addition to the side surface of the microwell, around the circumference of the opening of the microwell and the bottom surface of the microwell. The convex-concave structure may be formed throughout the entire bottom of the cell culture vessel. However, considering the possibility that microscope observation is performed following containing the cell in the microwell, the convex-concave structure is preferably not formed on the bottom surface of the microwell. In the present invention, the expression "the convex-concave structure is not formed on the bottom surface of the microwell" means that the convex-concave structure is not formed on, for example, 80% or more, preferably 90% or more, more preferably 95% or more, particularly preferably 98% or more, and especially preferably about 100% of a region with respect to the total area of the bottom surface of the microwell. When the convex-concave structure is formed on the bottom surface of the microwell, the light refraction and transmission are reduced and the microscope observation of the cell contained is sometimes affected, hence not preferable. However, even in such a case, a convex-concave structure inevitably formed due to the production process, that is, a structure configured by finer convexes and concaves having the width and depth smaller than those of the convex-concave structure of the present invention, is not excluded.

The bottom surface of the microwell is preferably a smooth surface. In the present invention, the expression "the bottom surface of the microwell is a smooth surface" means that, for example, 80% or more, preferably 90% or more, more preferably 95% or more, particularly preferably 98% or more, and especially preferably about 100% of a region with respect to the total area of the bottom surface of the microwell is a smooth surface. For example, in the case where the bottom surface of the microwell has a bottom surface width to be described below, for example, a region of a circle having a diameter of 0.1 mm or more, preferably 0.12 mm or more, and more preferably 0.15 mm or more has a smooth surface. The diameter of the above circle can also be defined to be X + m (X herein represents the maximum diameter of a cell to be observed). Here, m is preferably 0.01 mm or more, and further preferably 0.02 mm or more. Further, in the present invention, "the smooth surface" means the bare minimum surface roughness. For example, the surface roughness of the bottom surface of the microwell has preferably a maximum height (Ry) of 3 µm or less, more preferably Ry of 1 µm or less, and further preferably Ry of 0.5 µm or less. In the present specification, the maximum height (Ry) means a value (in micrometer unit) obtained by taking out only the reference length in the direction of the average line from the roughness curve and measuring the distance between the top line and the bottom line of the taken out part in the direction of the depth magnification of the roughness curve. According to the cell culture vessel of the present invention, a cell to be observed is contained in the microwell, cultured and observed. When observing the cell, an image of the cell is obtained typically using an observation device to be described below in the direction from the bottom surface or the direction from the opening of the microwell. At this operation, if there are finer convexes and concaves having the width and depth smaller than those of the convex-concave structure of the present invention, in other words, many fine peaks and valleys on the bottom surface of the microwell, the surface roughness of the bottom surface becomes significant and, an unclear image of the cell may be resulted. For this reason, when the bottom surface of the microwell has a smooth surface with the feature described above, a clear image can be obtained at the time of observing the cell.

Note that the surface roughness of the bottom surface of the microwell can be reduced by, for example, increasing machining accuracy of a die using a treatment such as polishing the surface of the die used when producing the cell culture vessel of the present invention.

The shape of the convex-concave structure is not particularly limited as long as the shape facilitates the bubble removal, and examples include structures configured by at least two linear convex parts and concave parts and structures configured by at least two dot projections and/or at least two dot recessions.

Examples of the convex-concave structure configured by at least two linear convex parts and concave parts include structures configured by at least two linear convex parts continuously formed with spaces in between and structures configured by at least two linear concave parts continuously formed with spaces in between. In the structures configured by at least two linear convex parts continuously formed with spaces in between, the space part can be regarded as the concave part and thus, in other words, the structure is substantially configured by at least two linear concave parts continuously formed with spaces in between. Hereinbelow, in the description, the convex part is regarded as the line and the concave part is regarded as the space. The linear convex part is preferably formed from the bottom surface of the microwell in the direction toward the opening. For example, the lines arranged on a plane parallel to the bottom surface of the microwell, that is, the ring-shaped lines arranged along the microwell side surface on a plane parallel with the bottom surface of the microwell, may also be considered, but such an arrangement is difficult to process when producing the microwell by injection molding, or the like, and is presumed to have a poor bubble removable effect. When the side surface of the microwell is perpendicular to the bottom surface, the linear convex part is also preferably formed perpendicularly to the bottom surface but may be somewhat inclined along the microwell side surface. In the instance, the inclination is preferably 30° or less to the perpendicular direction. The inclination herein, in the development view of the microwell side surface, can be defined to be an angle against the line, when which is perpendicular.

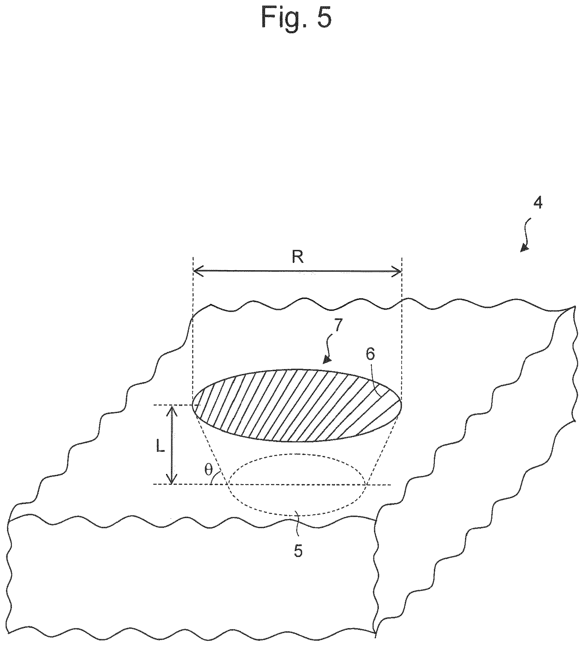

When the side surface of the microwell is inclined to the bottom surface, for example, as shown in Figures 3 and 4, the linear convex part is also inclined to the bottom surface at the same angle as the microwell side surface but preferably is formed to be on the intersection of a plane passing the centroid of the bottom surface and perpendicular to the bottom surface and the side surface. Similarly in this case, for example, as shown in Figure 5, the line may be somewhat inclined along the microwell side surface, and the inclination is preferably 30° or less to the perpendicular direction. The inclination, in the development view of the microwell side surface, can be similarly defined to be an angle against the line, when which is not inclined (i.e., the line shown in Figure 3). The line and space may be a straight line or a curved line, but a straight line is preferable due to easy molding and a high bubble removable effect.

In the convex-concave structure configured by at least two linear convex parts and concave parts, both of the at least two linear convex parts preferably have the substantially same width and height. Further, both of the at least two concave parts corresponding to the spaces preferably have the substantially same width and height (depth).

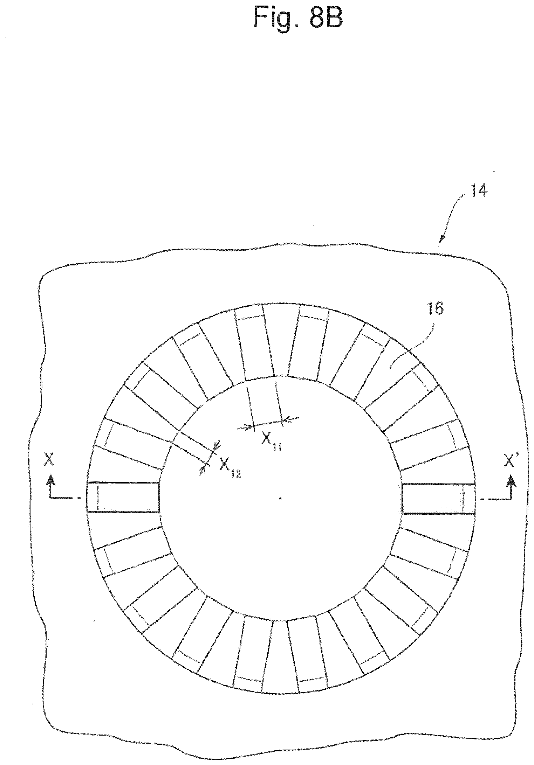

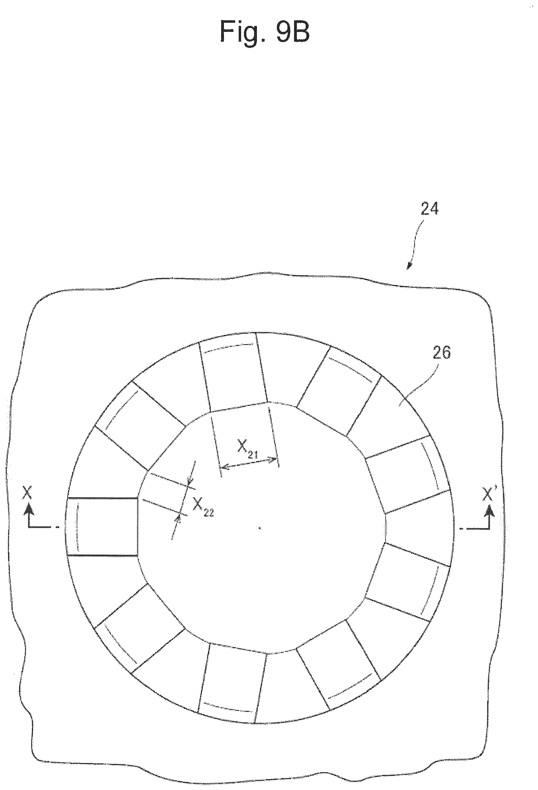

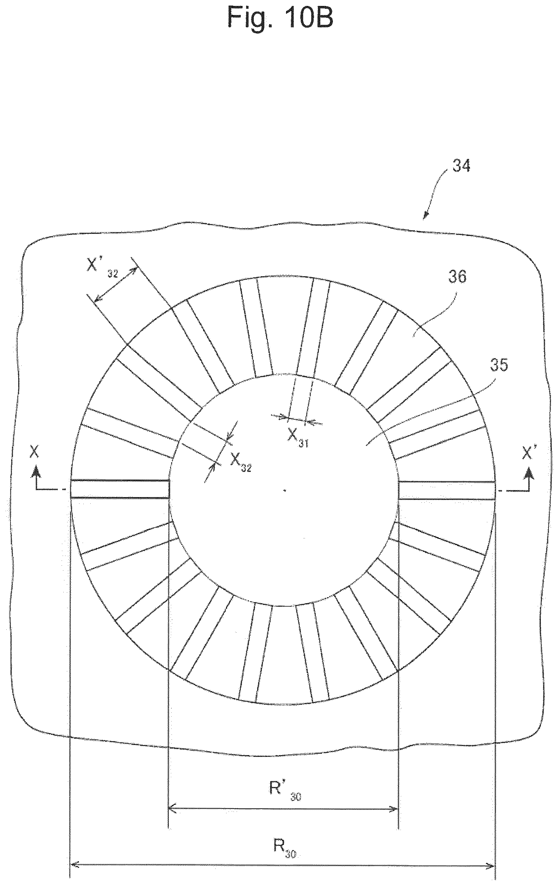

In the convex-concave structure configured by at least two linear convex parts and concave parts, the width of the linear convex part (e.g., X₁ in Figure 4, X₁₁ in Figure 8B, X₂₁ in Figure 9B, and X₃₁ in Figure 10B) and the width of the concave part corresponding to the space (e.g., X₂ in Figure 4, X₁₂ in Figure 8B, X₂₂ in Figure 9B, and X₃₂ in Figure 10B) range from 1/3140 to 1/4.5, preferably 1/314 to 1/4.5, further preferably 1/165 to 1/15, and particularly preferably 1/82.5 to 1/15 of the length of the outer peripheral portion of the opening.

In the linear convex-concave structure, the width of a single linear convex part and the width of a single concave part corresponding to a space are substantially constant, respectively. In this instance, the width of the linear convex part (e.g., X₁ in Figure 4, X₁₁ in Figure 8B, X₂₁ in Figure 9B, and X₃₁ in Figure 10B) is preferably 0.1 µm or more, more preferably 1 µm or more, further preferably 10 µm or more, particularly preferably 15 µm or more, and especially preferably 20 µm or more, and preferably 100 µm or less, more preferably 70 µm or less, and further preferably 50 µm or less, and preferably in a range from 1 to 70 µm, more preferably in a range from 10 to 70 µm, further preferably in a range from 15 to 70 µm, and particularly preferably in a range from 20 to 70 µm. The width of the concave part corresponding to the space (e.g., X₂ in Figure 4, X₁₂ in Figure 8B, X₂₂ in Figure 9B, and X₃₂ in Figure 10B) is preferably 0.1 µm or more, more preferably 1 µm or more, and further preferably 10 µm or more, and preferably 100 µm or less, more preferably 70 µm or less, and further preferably 50 µm or less, and preferably in a range from 1 to 70 µm, more preferably in a range from 10 to 70 µm, further preferably in a range from 15 to 70 µm, and particularly preferably in a range from 20 to 70 µm. Note that, in the cell culture vessel according to the present invention, the width of the linear convex part and the width of the concave part corresponding to the space can be determined, for example, by measuring widths of convex parts and widths of concave parts of a plurality of the convex-concave structures in randomly selected microwells using a 3D measurement laser microscope and calculating each of the average value of the measured values.

With the widths of the convex part and the concave part being a certain value or more, the likelihood of deteriorated processing and molding accuracy can be obviated. Conversely, with the above widths being a certain value or less, the difficulty in shaping caused by over-sized convex-concave structure with respect to the structure of the microwell can be obviated. Preferably, the width of the linear convex part and the width of the concave part are same. When the widths are same at the time of molding and processing, the resin flowability may be easily controlled and the processing accuracy may be improved.

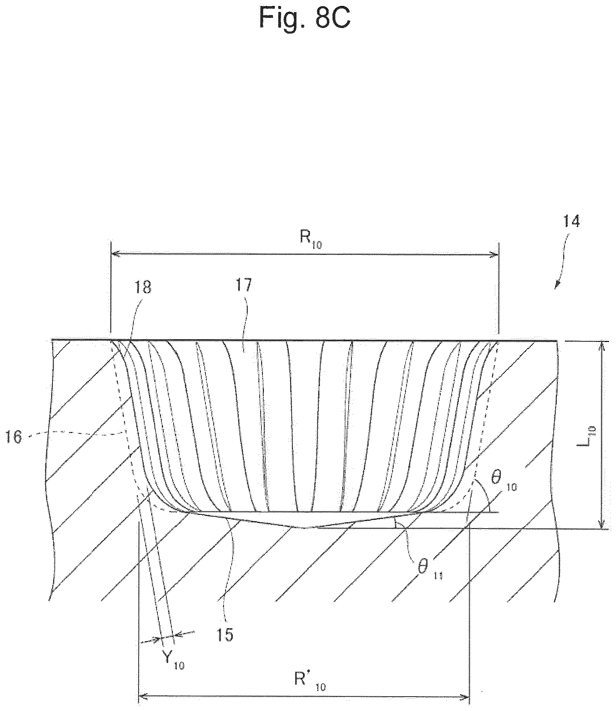

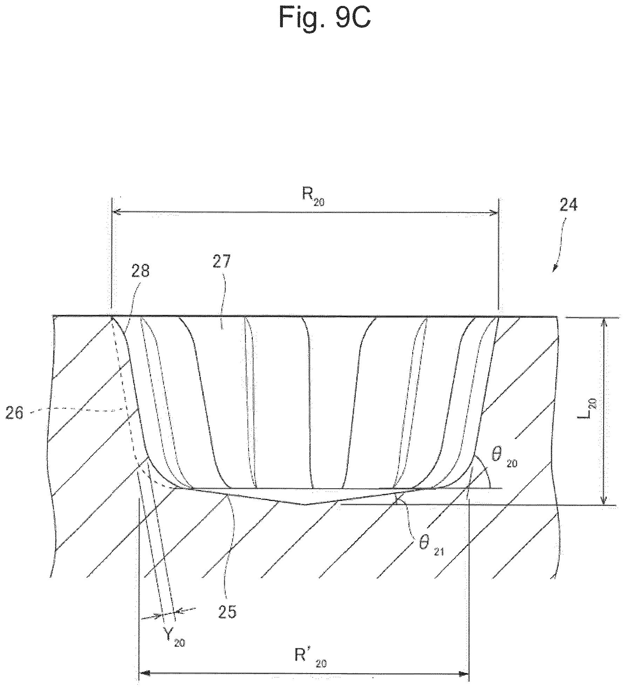

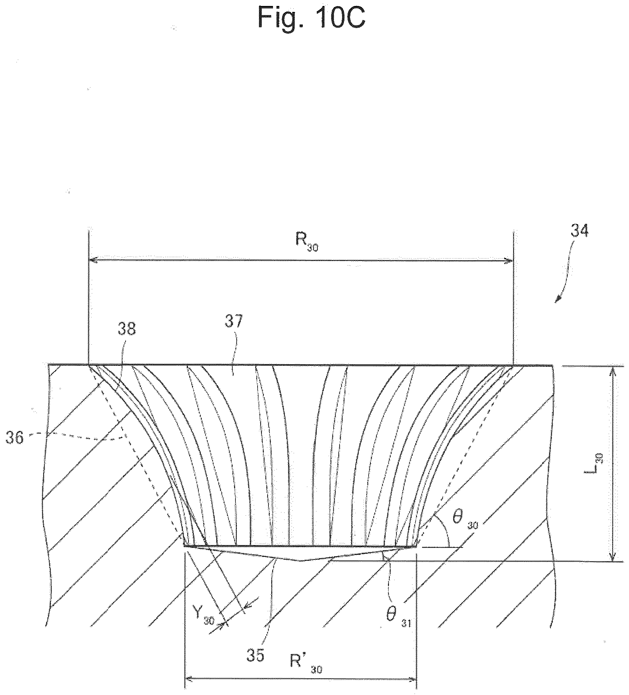

In the linear convex-concave structure, a cross-section of the linear convex part preferably has a square shape or an arc shape on a plane passing the centroid of the bottom surface of the microwell and orthogonal to a plane parallel with the bottom of the cell culture vessel. The centroid of the bottom surface of the microwell herein means the centroid on a figure obtained by projecting the bottom surface of the microwell onto a plane parallel with the bottom of the cell culture vessel of the present invention including the outer periphery of the bottom surface of the microwell. In the case where a cross-section of the linear convex part is square, the cross-section of the convex part is preferably a square having the height to be described below (e.g., Y in Figure 4, Y₁₀ in Figure 8C, and Y₂₀ in Figure 9C). In this case, the corners of the convex part may be rounded, in other words, may have a curvature. In the case where a cross-section of the linear convex part has an arc shape, the cross-section of the convex part has the height to be described below (e.g., Y₃₀ in Figure 10C), and the arc shape preferably has a curvature radius ranging from 100 to 500 µm, and preferably ranging from 200 to 400 µm. In the above case, the concave part lying between two linear convex parts can be the space part between the two linear convex parts. For this reason, the cross-section of the concave part on a plane passing the centroid of the bottom surface of the microwell and orthogonal to a plane parallel with the bottom of the cell culture vessel is the same as the cross-section of the side surface of the microwell.

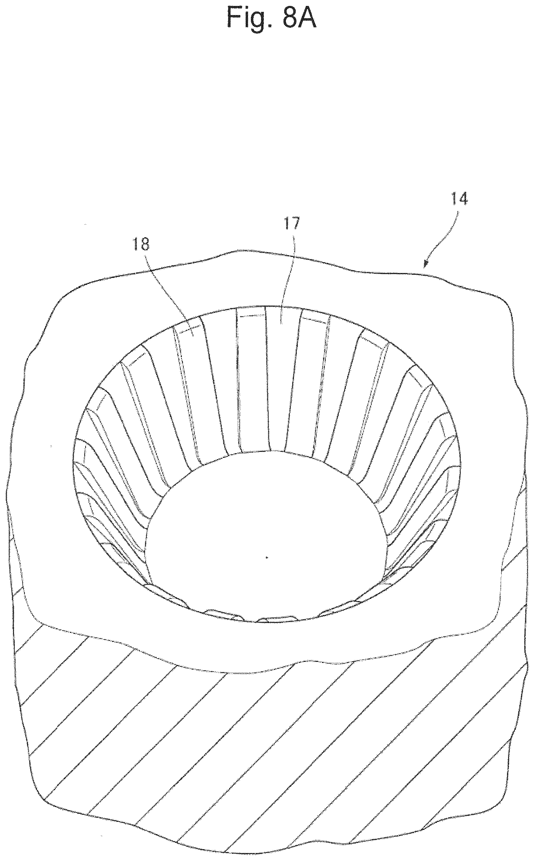

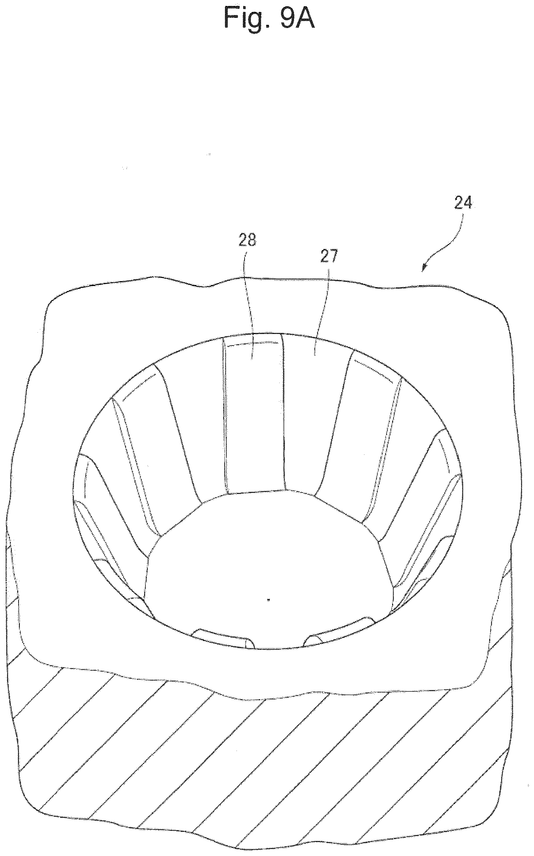

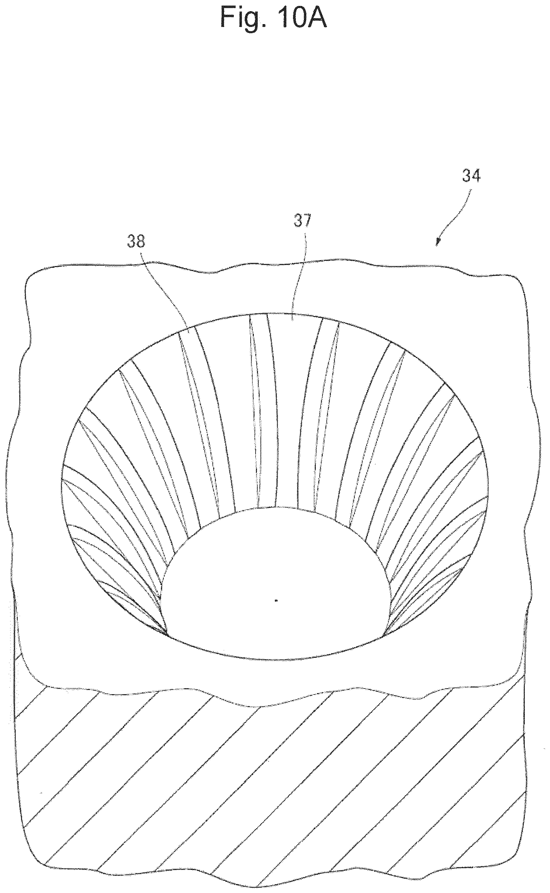

A height of the linear convex part with respect to the space part (concave part) (e.g., Y in Figure 4, Y₁₀ in Figure 8C, Y₂₀ in Figure 9C, and Y₃₀ in Figure 10C) is preferably 5 µm or more, and preferably 100 µm or less, more preferably 70 µm or less, and further preferably 50 µm or less, preferably in a range from 5 to 70 µm and further preferably in a range from 5 to 50 µm. In the linear convex-concave structure, when the height of each of the convex parts with respect to the concave parts is substantially fixed (e.g., in the case where the cross-section of the linear convex part is square shape as in the convex-concave structure 18 of the cell culture vessel shown in Figures 8A to C and the convex-concave structure 28 of the cell culture vessel shown in Figures 9A to C), the above values mean the perpendicular distance from the bottom surface of the concave part to the upper part of each of the convex parts (e.g., Y₁₀ in Figure 8C). In the linear convex-concave structure, when the height of each of the convex parts with respect to the concave parts is not fixed (e.g., the convex-concave structure 38 of the cell culture vessel shown in Figures 10A to C), the above values mean the maximum value of the perpendicular distance from the bottom surface of the concave part to the upper part of each of the convex parts, that is, the maximum height of each of the convex parts with respect to the concave parts (e.g., Y₃₀ in Figure 10C). The ratio of the height to the width of the linear convex part (e.g., Y/X₁ in Figure 4, Y₁₀/X₁₁ in Figures 8B and C, Y₂₀/X₂₁ in Figures 9B and C, Y₃₀/X₃₁ in Figures 10B and C) is preferably 0.05 or more, more preferably 0.1 or more, further preferably 0.2 or more, and particularly preferably 0.3 or more, and preferably 1.5 or less, and more preferably 1.0 or less, and preferably in a range from 0.05 to 1.5, more preferably in a range from 0.1 to 1.5, further preferably in a range from 0.2 to 1.5, particularly preferably in a range from 0.2 to 1.0, and especially preferably in a range from 0.3 to 1.0. Note that, in the cell culture vessel according to the present invention, a height of the convex part with respect to the concave part can be determined, for example, by measuring heights of a plurality of convex parts in randomly selected microwells using a 3D measurement laser microscope and calculating an average value of the measured values. Further, a ratio of the height to the width of the convex part can be calculated using the values of width and height of the convex part determined by the method described above.

With the height being a certain value or more, the likelihood that the wettability is not enhanced and thereby bubbles remain can be obviated. Further, with the height being a certain value or less, the likelihood that the processing and molding accuracy to the microwell structure is deteriorated and the wettability enhancement effect is reduced and thereby residual bubbles are caused can be obviated.

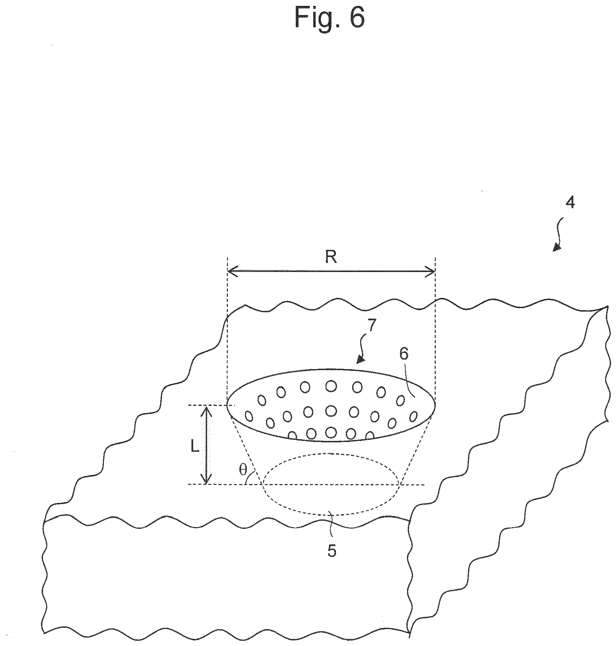

In the convex-concave structure configured by at least two dot projections and/or at least two dot recessions, the shape of dot is not particularly limited. Examples of the shape of the dot projection and recession include cones, frustums, cylinders, and random anisotropic projections and recessions. Conical, frustum and cylindrical projections and recessions are preferable from a standpoint of processability. For example, Figure 6 shows an enlarged perspective view of the microwell with cylindrical projections formed on the side surface thereof.

In the cases of conical projection and frustum projection, the shape of projection in which the projection becomes narrower toward its end is preferable from a standpoint of processability, and in the cases of conical recession and frustum recession, the shape of recession in which the opening width becomes wider the opening side is preferable from a standpoint of processability. The width of conical projection, frustum projection, and cylindrical projection and the opening width of conical recession, frustum recession, and cylindrical recession are preferably 0.1 µm or more, more preferably 1 µm or more, and further preferably 5 µm or more, and preferably 50 µm or less, more preferably 25 µm or less, and further preferably 10 µm or less.

With the width of the projection and the opening width of the recession being a certain value or more, the likelihood of deteriorated processing and molding accuracy can be obviated. Further, with the above widths being a certain value or less, the difficulty in shaping caused by the over-sized concave part with respect to the structure of the microwell can be obviated.

The width of the projection means a maximum value of the maximum diameter at the sectional figure perpendicular to the axis of the projection, the widths of the conical projection, frustum projection, and cylindrical projection means a maximum diameter of the bottom surface of a cone and a cylinder or the lower bottom of a frustum, and when the bottom surface or the lower bottom is circle, it means a diameter. The opening width of the recession means a maximum diameter of the figure at the opening of the recession, and when the figure of the opening is circle, it means a diameter.

The height of the dot projection and the depth of the dot recession are preferably 0.1 µm or more, more preferably 1 µm or more, and further preferably 5 µm or more, and preferably 50 µm or less, more preferably 25 µm or less, and further preferably 10 µm or less. The ratio of the projection height to the projection width (height/width) and the ratio of the recession depth to the recession opening width (depth/opening width) are preferably 0.1 or more, more preferably 0.3 or more, and preferably 1.5 or less, more preferably 1 or less.

With the projection height and the recession depth being a certain value or more, the likelihood of deteriorated processing and molding accuracy can be obviated. Further, with the above height and depth being a certain value or less, the difficulty in shaping caused by the over-sized projecting part and recessed part with respect to the structure of the microwell can be obviated. Furthermore, with the ratio of heights being a fixed value or less, when molding the shapes of recession and projection on the side surface by injection molding, or the like, the difficult detachment and the shape breakage at the time of perpendicularly peeling from a die can be obviated.

The pitch of the dot projections and the pitch of the dot recessions are preferably 0.1 µm or more, more preferably 1 µm or more, and further preferably 5 µm or more, and preferably 50 µm or less, more preferably 25 µm or less, and further preferably 10 µm or less.

With these pitches being a certain value or more, the likelihood of deteriorated processing and molding accuracy can be obviated. Further, with these pitches being a certain value or less, the difficulty in shaping caused by the over-sized projecting part and recessed part with respect to the structure of the microwell can be obviated.

The pitch of the dot projections is the space between two projections adjacent to each other and means a distance between the centers of these projections. The center of the projection herein is the centroid of the figure at the top end part of the projection. The pitch of the dot recessions is the space between two recessions adjacent to each other and means a distance between the centers of these recessions. The center of the recession herein is the centroid of the figure at the opening of the recession. The pitch typically means an average pitch, the average pitch for a certain projection means an average value calculated from the pitches among all adjacent projections thereto, and that for a certain recession means an average value calculated from the pitches among all adjacent recessions thereto.

The dot projections and dot recession may be intermingled, but from a standpoint of processability, it is preferred that either only the dot projections or only the dot recessions be formed, and it is more preferred that only the dot projections be formed. When the projection and the recession are intermingled, die-and-mold and molding process become difficult which may cause poor shape accuracy and fail to achieve the desired effect of enhanced wettability.

The method for forming the convex-concave structure on the side surface of the microwell is not particularly limited. When the cell culture vessel with a microwell is produced by injection molding, the convex-concave structure on the side surface can also be easily produced by injection molding at the time of molding the microwell. Alternatively, various methods utilizing etching may also be used.

When the convex-concave structure is formed on the side surface of the microwell, hydrophilicity can be enhanced to the wettability of the conventional resins. For example, the contact angle value of water or alcohol becomes smaller resulting in better slippage of droplets. Further, when the convex-concave structure is formed on the side surface of the microwell, bubbles are more easily removed than those having no convex-concave structure, whereby the operability is improved. Furthermore, when the shape and size of the convex-concave structure formed on the side surface are limited, the productivity of the microwell can be increased.

In addition to the convex-concave structure, when the side surface of the microwell has a hydrophilic surface, the bubble removable performance can even be improved. Although it is sufficient if at least the side surface of the microwell is a hydrophilic surface, preferably the bottom surface of the microwell is also a hydrophilic surface, more preferably the circumference of the microwell is also a hydrophilic surface, and further preferably the entire bottom of the cell culture vessel is a hydrophilic surface. The cell culture vessel may entirely have a hydrophilic surface. In the present invention, the expression "the side surface of the microwell is a hydrophilic surface" means that, for example, 80% or more, preferably 90% or more, more preferably 95% or more, particularly preferably 98% or more, and especially preferably about 100%, of a region with respect to the total area of the side surface of the microwell is a hydrophilic surface. In the present invention, the expression "the bottom surface of the microwell is a hydrophilic surface" means that, for example, 80% or more, preferably 90% or more, more preferably 95% or more, particularly preferably 98% or more, and especially preferably about 100%, of a region with respect to the total area of the bottom surface of the microwell is a hydrophilic surface. The hydrophilic surface encompasses surfaces, which are made hydrophilic when materials for composing the vessel and microwell are hydrophilic and surfaces, which are made hydrophilic by subjecting the surfaces of the vessel and microwell to hydrophilization treatment. When the microwell circumference is a hydrophilic surface in addition to the side surface, fluid flowability is even more increased and bubbles is less likely to be left. When hydrophilization treatment is carried out, the entire area is treated all at once because highly productive and more stable treatment can be achieved.

Hydrophilization treatment can be carried out by a routine method used in the art and thus is not particularly limited. Examples include plasma treatment, coating treatment, UV irradiation treatment, EB irradiation treatment, and graft polymerization treatments of hydrophilic polymer or the like to the surface, but plasma treatment is preferable from a standpoint of treating the entire area uniformly even when a shape to be treated has a three-dimensionally minute and complicated structure. Note that hydrophilization treatment is preferably carried out after the cell culture vessel with a microwell is molded.

Examples of the plasma treatment include plasma treatment using an inert gas. Examples of the inert gas include rare gases such as helium (He), neon (Ne), argon (Ar) and xenon (Xe), nitrogen (N₂), and oxygen (O₂). Plasma treatment using an oxygen gas or an argon gas is preferable from a standpoint of retaining hydrophilicity for an extended period of time on the surface to be treated. Low-temperature plasma treatment generated under the atmospheric pressure is also preferable from a standpoint of preventing the surface deterioration (decomposition and etching), or the like.

The pressure at the time of plasma generation in a vacuum environment is preferably 1 Pa or less, and preferably 0.2 Pa or more, and more preferably 0.4 Pa or more. The total gas flow rate at the time of plasma generation is not particularly limited but typically 5 to 50 ml/min, and preferably 10 to 30 ml/min. With a flow rate being in the above range, a pressure deviation at the time of plasma generation can be inhibited. The time for plasma irradiation is preferably 10 minutes or less, more preferably 5 minutes or less, and further preferably 1 minute or less, and preferably 2 seconds or more, and more preferably 10 seconds or more. When the irradiation time is too short, the plasma treatment effect may not be achieved. The electric power applied to the plasma generation portion is preferably 100 W or more, more preferably 200 W or more, and further preferably 300 W or more, and preferably 500 W or less. With the electric power being a certain value or more, plasma is stabilized and thus variability can be inhibited.

Further, at the time of plasma generation under the atmospheric pressure, the total gas flow rate is not particularly limited and for an argon gas, for example, is typically 1 to 10 1/min, and preferably 3 to 7 1/min. With a flow rate being in the above range, a pressure deviation at the time of plasma generation can be inhibited. The time for plasma irradiation is preferably 10 minutes or less, more preferably 5 minutes or less, and further preferably 1 minute or less, and preferably 2 seconds or more, and more preferably 10 seconds or more. With the irradiation time being certain time or more, the plasma treatment effect can be definitely imparted. When the plasma irradiation conditions are as described above both in a vacuum environment and under the atmospheric pressure, hydrophilization treatment with more stability and longer-term retention can be achieved.

Examples of the coating treatment include coating treatments using a hydrophilic polymer. The hydrophilic polymer refers to a polymer, which contains a carbon component and includes a hydrophilic functional group on the main chain or a side chain thereof. The hydrophilic polymer is preferably a water soluble polymer, which has water solubility and water swellability and includes a carbon-oxygen bond. The hydrophilic polymer may be those permanently having water solubility and water swellability or those showing water solubility and water swellability caused by a predetermined stimulation such as light, temperature, or pH.

Specific examples of the hydrophilic polymer include hydrogel polymers such as polyalkylene glycol, polyvinyl pyrrolidone, polypropylene glycol, polyvinyl alcohol, polyethylene imine, polyallyl amine, polyvinyl amine, polyvinyl acetate, polyacrylic acid, and poly(meth)acryl amide, poly-N-isopropylacrylamide, copolymers of these polymers and other monomers, and graft polymers. Of these, polyalkylene glycol is commercially available in various molecular weights and has good biocompatibility, hence can be preferably used. Specific examples of polyalkylene glycol include polyethylene glycol (PEG), polypropylene glycol, and a copolymer of ethylene glycol and propylene glycol, with polyethylene glycol being particularly preferably used to be the hydrophilic polymer in the present invention.

When the vessel is formed using the hydrophilic materials such as the hydrophilic polymers described above, the surface can be a hydrophilic surface without hydrophilization treatment. Examples of other hydrophilic material include polyacrylonitrile, nylon 6, polyethylene terephthalate, polymethyl acrylate, polymethyl methacrylate and phenol resins.

The hydrophilic surface refers to a surface having a water contact angle of 70° or less. The water contact angle of the hydrophilic surface is preferably 60° or less, more preferably 50° or less, further preferably 45° or less, and particularly preferably 40° or less, and preferably 10° or more. When a water contact angle is within the above range, the hydrophilicity is considered sufficient and accordingly the bubble removable effect is expected to be high. Note that the water contact angle means a water contact angle measured at 23°C.

The opening of the microwell has an opening width capable of containing a cell. The opening width of the opening of the microwell herein means a length of the smallest diameter of a figure configured by the outer periphery of the opening of the microwell. Consequently, when the outer periphery of the opening of the microwell is circle, the opening width is equal to a diameter of the circle, and the diameter must be larger than the maximum size of a cell to be cultured. When a fertilized egg is cultured using the cell culture vessel of the present invention, it is desirable to culture the egg to the blastocyte stage. For this reason, the diameter of the circular opening is desirably larger than the maximum size of the cell at the blastocyte stage. The opening width of the opening of the microwell (e.g., R in Figure 3 and Figure 4, R₁₀ in Figure 8C, R₂₀ in Figure 9C, R₃₀ in Figure 10C, and the diameter in the case where the outer periphery of the opening of the microwell is circle) is preferably 0.1 mm or more and 1 mm or less. The above opening width is preferably 0.25 mm or more, more preferably 0.26 mm or more, and further preferably 0.27 mm or more, and preferably below 0.7 mm, and further preferably below 0.45 mm. Further, the above opening width of the opening of the microwell can also be defined to be X + m (X herein represents the maximum diameter of a cell). m herein is preferably 0.01 mm or more, and further preferably 0.02 mm or more.

The depth of the microwell means a depth measured perpendicularly to the deepest part from the opening of the microwell (e.g., L in Figure 3, L₁₀ in Figure 8C, L₂₀ in Figure 9C, and L₃₀ in Figure 10C), and is preferably 0.1 mm or more and 0.5 mm or less. The depth of the microwell must be designed to hold a cell within the microwell without fail because the cell moves during transport of the culture vessel or cell division, or the like, and is likely to get out from the range of the microwell when the microwell is too shallow. For example, for holding a cell within the microwell, the depth is preferably 1/3 or more, and further preferably 1/2 or more, of the maximum diameter of the cell. On the other hand, when the depth is too deep, it becomes difficult to introduce a culture medium and a cell into the microwell. Thus, the depth must be suitably designed to have a value not too deep while the cell is held in the microwell. For example, the upper limit of the depth can be designed to be three times or less with respect to the opening width of the opening of the microwell. Further, for easy introduction of a culture medium, the depth is preferably equal to or less, and particularly preferably 1/2 or less, of the opening width of the microwell.

The bottom surface of the microwell may be, at the disposition when the cell culture vessel is used, a horizontal plane or may have an upward inclined plane toward the outer periphery from the lowest point. The bottom surface of the microwell is preferably configured to have a downward inclined plane to the surface of the bottom of the cell culture vessel, more preferably configured to have an inclined plane which is downward to the surface of the bottom of the cell culture vessel and at least a part of which has a straight-line inclination, further preferably configured to be an inverted conical shape having a downward inclined plane to the surface of the bottom of the cell culture vessel, and particularly preferably configured to be an inverted straight conical shape having a downward inclined plane to the surface of the bottom of the cell culture vessel and having, as a central axis, a straight line passing the centroid of the bottom surface and orthogonal to a plane parallel with the bottom of the cell culture vessel. In the above inclined planes, the lowest point is preferably disposed in a region other than the outer periphery of the bottom surface of the microwell, and more preferably disposed on the straight line passing the centroid of the microwell and orthogonal to a plane parallel with the bottom of the cell culture vessel. The centroid of the bottom surface of the microwell herein means the centroid on a figure obtained by projecting the bottom surface of the microwell onto a plane parallel with the bottom of the cell culture vessel of the present invention including the outer periphery of the bottom surface of the microwell. When the bottom surface has an inclined plane, a downward inclination angle to the surface of the bottom of the cell culture vessel, for example, an inclination angle to a horizontal plane at the disposition when the cell culture vessel is used (e.g., θ₁₁ in Figure 8, θ₂₁ in Figure 9, and θ₃₁ in Figure 10), is preferably 45° or less, more preferably 30° or less, particularly preferably 10° or less, especially preferably in a range from 5 to 10°, and still especially preferably about 7°. When the bottom surface has an inclined plane, a cell can be retained at the central part (the deepest part) of the microwell, in other words, the cell does not shift from the central position even when somewhat swayed, whereby the observation using a microscope is easy to conduct without looking for the cell, hence advantageous.

The bottom surface of the microwell has a bottom surface width capable of containing a cell. The bottom surface width of the bottom surface of the microwell herein means a length of the smallest diameter of a figure formed by the connecting part between the bottom surface and the side surface of the microwell (e.g., R'₁₀ in Figure 8C, R'₂₀ in Figure 9C, and R'₃₀ in Figure 10C). Consequently, when the bottom surface of the microwell is circle, the bottom surface width is equal to the diameter of the circle, and the diameter must be larger than the maximum size of a cell to be cultured. As described below, when the connecting part between the bottom surface and the side surface of the microwell has a curvature, the bottom surface width means a length of the smallest diameter of a figure formed by the connecting part when given that the connecting part between the bottom surface and the side surface has no curvature (e.g., R'₁₀ in Figure 8C and R'₂₀ in Figure 9C). When a fertilized egg is cultured using the cell culture vessel of the present invention, it is desirable to culture the egg to the blastocyte stage. For this reason, the bottom surface width of the bottom surface of the microwell is desirably larger than the maximum size of the cell at the blastocyte stage. Further, the bottom surface width of the bottom surface of the microwell is preferably 0.1 mm or more and 1 mm or less. The above width is preferably 0.25 mm or more, more preferably 0.26 mm or more, and further preferably 0.27 mm or more, and preferably below 0.7 mm, and further preferably below 0.45 mm. Further, the above bottom surface width of the bottom surface of the microwell can also be defined to be X + m (X herein represents the maximum diameter of a cell). m herein is preferably 0.01 mm or more, and further preferably 0.02 mm or more.

The side surface of the microwell may be, at the disposition when the cell culture vessel is used, perpendicular to a horizontal plane or may be inclined. The side surface of the microwell is preferably inclined against a straight line orthogonal to the surface of the bottom of the cell culture vessel. More specifically, the side surface of the microwell is preferably inclined to the surface of the bottom of the cell culture vessel at the cross-section on a plane passing the centroid of the bottom surface of the microwell and orthogonal to a plane parallel with the bottom of the cell culture vessel. The centroid of the bottom surface of the microwell herein means the centroid on a figure obtained by projecting the bottom surface of the microwell onto a plane parallel with the bottom of the cell culture vessel of the present invention including the outer periphery of the bottom surface of the microwell. When the side surface is inclined, an inclination angle of the side surface of the microwell to a plane parallel with the surface of the bottom of the cell culture vessel, at the cross-section on a plane passing the centroid of the bottom surface of the microwell and orthogonal to a plane parallel with the bottom of the cell culture vessel, for example, an angle to a horizontal plane at the disposition when the cell culture vessel is used (e.g., θ in Figure 3, θ₁₀ in Figure 8C, θ₂₀ in Figure 9C, and θ₃₀ in Figure 10C), is preferably 45° or more, more preferably 60° or more, particularly preferably 70° or more, and preferably below 90°, more preferably 85° or less, and particularly preferably 80° or less, and preferably in a range from 45 to 85°, and more preferably in a range from 60° to 80°. Alternatively, an inclination angle at the side surface of the microwell against a straight line orthogonal to the surface of the bottom of the cell culture vessel, for example, an angle to a perpendicular straight line at the disposition when the cell culture vessel is used (e.g., 90°-θ in Figure 3, 90°-θ₁₀ in Figure 8C, 90°-θ₂₀ in Figure 9C, and 90°-θ₃₀ in Figure 10C), is preferably exceeding 0°, more preferably 5° or more, particularly preferably 10° or more, and preferably 45° or less, more preferably 30° or less, and particularly preferably 20° or less, and preferably in a range from 5 to 45°, and more preferably in a range from 10° to 30°. When the side surface is inclined at such an angle θ (e.g., θ₁₀ in Figure 8C, θ₂₀ in Figure 9C, and θ₃₀ in Figure 10C), bubbles can be prevented from remaining in the microwell when a culture medium is added thereto. The cell is also prevented from jumping out of the microwell and can be held within the microwell even when some vibrations and sways are caused.

Particularly preferably, in the cell culture vessel of the present invention,
the bottom surface of the microwell has an inverted conical shape with a downward inclined plane to the surface of the bottom of the cell culture vessel,
the side surface of the microwell is inclined against a straight line orthogonal to the surface of the bottom of the cell culture vessel, and
in the convex-concave structure configured by at least two linear convex parts and concave parts, the cross-section of the linear convex part has a square shape or an arc shape on a plane passing the centroid of the bottom surface of the microwell and orthogonal to a plane parallel with the bottom of the cell culture vessel. When the above shape is achieved, the cell culture vessel of the present invention can substantially prevent bubbles from remaining in the microwell over an extended period of time when a culture medium is added. The cell is also prevented from jumping out of the microwell and can be held within the microwell even when some vibrations and sways are caused.

It is preferable that the connecting part between the bottom surface and the side surface and the opening end of the microwell, and, in the convex-concave structure configured by at least two linear convex parts and concave parts, the connecting part between the convex part and the bottom surface and the end portion at the opening end side of the convex part, be rounded, that is, it is preferable to have a curvature. In the instance, the curvature is preferably 0.01 mm or more, more preferably 0.02 mm or more, and preferably 0.1 mm or less, and more preferably 0.07 mm or less, respectively. When the connecting part between the bottom surface and the side surface and the opening end of the microwell, and, in the convex-concave structure configured by at least two linear convex parts and concave parts, the connecting part between the convex part and the bottom surface and the end portion at the opening end side of the convex part have a curvature, effects such as improved operability due to easier bubble removal and an increase in yield by an easier injection molding process using plastic are expected.

The size of the cell culture vessel is not particularly limited but preferably used are those with a circular opening and an opening width (e.g., r in Figure 2) of preferably 30 to 60 mm, and particularly 35 mm. The size is about the same as the petri dish conventionally used for cell culture. Vessels having such a size are preferable because they are easily produced from the common petri dish and likely to be adoptable to existing culture apparatuses or the like.

At least one, preferably four or more, preferably six or more, and more preferably eight or more of the microwell are formed adjacent to each other on the bottom of the cell culture vessel. A plurality of microwells need to be formed adjacent to each other, but microwells not adjacent to each other may further be formed separately. A group of a plurality of microwells formed adjacent to each other may be disposed in several groups, and these groups may not be adjacent to each other. The pitch between the adjacent microwells is 1 mm or less, preferably 0.7 mm or less, and further preferably 0.45 mm or less. An observation device, equipped with a 1/2-inch CCD element and an objective lens with magnifications of x 4, x 10, and x 20, is commonly used. The observable field with such an observation device, when an objective lens with a magnification of x 4 is selected, is 1.6 mm x 1.2 mm, and it is preferable to design so that at least four microwells are included in the observation field. However, the above pitch varies depending on the kind of cell to be contained. When the microwells are closely disposed with the pitch described above, many cells can be simultaneously cultured while individually controlled and further an image including many cells can be obtained at a time because many cells are included in a single field of a microscope.

The pitch between the microwells is a distance between the centers of the microwells adjacent to each other. The center of the microwell is the centroid of a figure formed by the outer periphery of the opening of the microwell and, when the outer periphery is circle, refers to the center of the circle. The pitch between the microwells typically means an average pitch, and the average pitch for a certain microwell means an average value calculated from the pitches of all the microwells adjacent thereto.

A plurality of microwells formed adjacent to each other, for example, as shown in Figure 1 and Figure 2, may be set apart by an inner wall surrounding them from other parts in the culture vessel. In the present embodiment, each group of the adjacent microwells is surrounded by the inner wall. When a plurality of groups of microwells are disposed on the bottom surface of the cell culture vessel, each group is preferably surrounded by the inner wall. Typically, for culturing a fertilized egg, or the like, a droplet of a culture medium containing the fertilized egg is formed on the culture vessel and the culture medium is prevented from being dried by covering the droplet with an oil. When a group of a plurality of microwells formed adjacent to each other is surrounded by the inner wall, the culture medium is enclosed therein to form a stable droplet and the dispersion of the culture medium can be prevented. The case where the culture medium is covered by an oil such as a mineral oil is also the same.

The material of a cell culture vessel is not particularly limited. Specific examples thereof include inorganic materials such as metal, glass and silicon; and organic materials such as plastics (for example, a polystyrene resin, a polyethylene resin, a polypropylene resin, an ABS resin, nylon, an acrylic resin, a fluorine resin, a polycarbonate resin, a polyurethane resin, a methylpentene resin, a phenol resins, a melamine resin, an epoxy resin, and a vinyl chloride resin). The cell culture vessel can be produced by a method known to those skilled in the art. For example, a culture vessel is formed of a plastic material by a customary molding method, for example, injection molding.

The cell culture vessel is preferably subjected to a surface hydrophilic treatment such as a plasma treatment in order to prevent nonspecific adhesion of cultured cells and inhomogeneity of culture medium drop by surface tension. The number of bacteria (bioburden number) attached to a vessel manufactured is preferably 100 cfu/vessel or less. Furthermore, it is preferable that a sterilization treatment such as γ sterilization is applied.

To the cell culture vessel, a surface treatment or surface coating which can facilitate growth of fertilized eggs may be applied. Particularly, when fertilized eggs are co-cultured with cells of other organs (for example, endometrial membrane cells or fallopian tube epithelial cells) in order to facilitate the growth of the fertilized eggs, these cells must be previously attached to a culture vessel. In such a case, it is advantageous that the surface of the culture vessel is coated with a cell-adhesive material.

Examples of a desired cell for culture include, but are not particularly limited to, a fertilized egg, an egg cell, an ES cell (embryonic stem cell) and iPS cell (induced pluripotent stem cell). The egg cell means an unfertilized egg cell, and includes an immature oocyte and a mature oocyte. The fertilized egg starts cleavage after fertilization, increases in cell number, like a 2-cell stage to a 4-cell stage and an 8-cell stage and develops into morula and blastocyst. Examples of the fertilized egg include the early embryos such as a 2-cell embryo, a 4-cell embryo and an 8 cell embryo, morula and blastocyst (including early blastocyst, expanded blastocyst and hatched blastocyst). The blastocyst means an embryo consisting of external cells having a potential of forming the placenta and inner cell mass having a potential of forming an embryo. The ES cell means an undifferentiated pluripotent or totipotent cell obtained from the inner cell mass of the blastocyst. The iPS cell means a somatic cell (principally, fibroblast) having pluripotency (like ES-cell), which is acquired by introducing several types of genes (transcription factor) into a somatic cell. More specifically, examples of the cell include a group of cells such as a fertilized egg and blastocyst.

The cell culture vessel of the present invention is suitable for culturing preferably mammalian cells and avian cells and particularly mammalian cells. The mammal means a warm-blooded vertebrate. Examples thereof include primates such as a human and a monkey; rodents such as a mouse, a rat and rabbit; pet animals such as a dog and a cat; and farm animals such as a cow, a horse and a pig. The cell culture vessel of the present invention is particularly suitable for culturing a human fertilized egg.

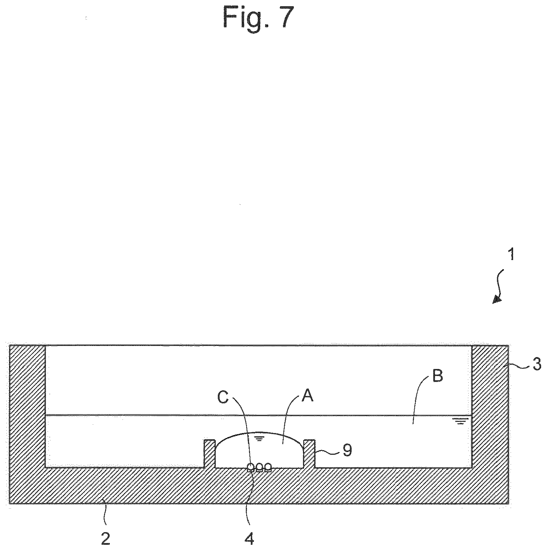

Usually, culture medium A is added so as to cover the micro well and oil B is added so as to cover the culture medium and then cell C is added to the culture medium. These operations are carried out usually by use of tools such as a pipette or a glass capillary. Since the cell culture vessel of the present invention has a large opening, these operations can be relatively easy carried out (Figure 7).

Culture is usually performed by placing the cell culture vessel in an incubator which brings an ambient atmosphere containing a gas required for growth and maintenance of a cultured cell and constant ambient temperature. Examples of the requisite gas include water vapor, free oxygen (O₂) and carbon dioxide (CO₂). The pH of the culture medium can be stabilized within a certain period of time by controlling ambient temperature and CO₂ content. Stable pH can be obtained by stabilizing CO₂ content and temperature. The culture conditions such as temperature, gas and culture medium, can be controlled also by comparing the image of a cell during culture to a pre-stored image by use of an image comparison program.

For example, if a fertilized egg is cultured, usually whether the cultured fertilized egg is satisfactory and suitable or not for implantation into the uterus is determined. The determination may be automatically performed or manually by e.g., a microscope. In automatic determination of a cultured cell, the image of a cell in a culture vessel is captured by a microscope, photographed by a detection apparatus such as a CCD camera and subjected to a contour extraction process in which a portion corresponding to the cell is extracted from the image. The extracted cell image is analyzed by the image analysis apparatus to determine the quality of the cell. As the image contour extraction process, for example, a process described in JP Patent Publication (Kokai) No. 2006-337110 can be used.

### EXAMPLES

### <Comparative Example>

A polystyrene cell culture vessel with 25 (5 x 5) microwells formed on the bottom thereof was produced, wherein the microwell had a circular opening with a diameter of 335 µm, a circular bottom surface with a diameter of 285 µm, a depth of 150 µm, and a side surface was inclined at 80° (θ = 80°). 100 µL of water was added dropwise to each of the microwells. As a result, bubbles remained in all of the microwells. The bubbles were not easily removed and consequently the cell culture was not feasible.

### <Example 1>

The cell culture vessel produced in Comparative Example was hydrophilized by plasma treatment, stored for half a year, and measured for a water contact angle of the vessel surface and which was found to be 50°.

When 100 µL of water was added dropwise to each of the microwells, several bubbles remained in some microwells but were easily removed when the side surface of the vessel was lightly tapped. Thus, the culture was feasible in all of the microwells.

### <Example 2>

The cell culture vessel produced in Comparative Example was hydrophilized by plasma treatment, stored for two years, and measured for a water contact angle of the vessel surface and which was found to be 60°.

When 100 µL of water was added dropwise to each of the microwells, bubbles remained and were not removed in some microwells even when the side surface of the vessel was tapped.

### <Example 3>

A cell culture vessel with 25 (5 x 5) microwells formed on the bottom thereof was produced, wherein the microwell had a circular opening with a diameter of 335 µm, a circular bottom surface with a diameter of 285 µm, a depth of 150 µm, a structure configured by linear convex parts and concave parts with a width (X₁) of 20 µm and a depth (Y) of 5 µm on the side surface thereof, and the side surface thereof was inclined at 80° (θ = 80°).

100 µL of water was added dropwise to each of the microwells. As a result, bubbles remained in most of the microwells but were easily removed when the side surface of the vessel was lightly tapped. Thus, the culture was feasible in all of the microwells.

### <Example 4>

The cell culture vessel, produced in Example 3 and hydrophilized by plasma treatment, was stored for half a year and measured for a water contact angle of the vessel surface (on which the convex-concave structure was not formed) which was found to be 50°. To separately measure a water contact angle on a structure configured by linear convex parts and concave parts, a plate was produced wherein a structure as in the structure configured by linear convex parts and concave parts produced on the microwell side surface in Example 3 was molded on a horizontal surface, subjected to the same plasma treatment to be a sample, and measured for a water contact angle, and which was found to be 10° in those stored for half a year.

100 µL of water was added dropwise to each of the microwells but no single bubble remained. Thus, the culture was feasible in all of the microwells.

### <Example 5>

The cell culture vessel, produced in Example 3 and hydrophilized by plasma treatment, was stored for 2 years, and measured for a water contact angle of the vessel surface (on which the convex-concave structure was not formed) which was found to be 60°. Further, to separately measure a water contact angle on a structure configured by linear convex parts and concave parts, a plate to be a sample was produced with a structure as in the structure configured by linear convex parts and concave parts formed on the microwell side surface in Example 3 molded on a horizontal surface thereof, subjected to the same plasma treatment, and measured for a water contact angle, which was found to be 30° in those stored for 2 years.

100 µL of water was added dropwise to each of the microwells but no single bubble remained. Thus, the culture was feasible in all of the microwells.

### <Example 6>

The cell culture vessel, produced in Example 3 and hydrophilized by plasma treatment, was stored for 2 and a half years, and measured for a water contact angle of the vessel surface (on which the convex-concave structure was not formed), and which was found to be 70°. Further, to separately measure a water contact angle on a structure configured by linear convex parts and concave parts, a plate to be a sample was produced with a structure as in the structure configured by linear convex parts and concave parts produced on the microwell side surface in Example 3 molded on a horizontal surface thereof, subjected to the same plasma treatment, and measured for a water contact angle, which was found to be 50° in those stored for 2 and a half years.

When 100 µL of water was added dropwise to each of the microwells, several bubbles remained but were easily removed when the side surface of the vessel was lightly tapped.

The above results are collectively shown in the following Table 1.

**Table 1**

| | Hydrophilization treatment | Convex -concave structure | Storage period | Result |
|---|---|---|---|---|
| Comparative Example | - | - | - | Poor |
| Example 1 | ○ | - | A half year | Good |
| Example 2 | ○ | - | Two years | Fair |
| Example 3 | - | ○ | - | Good |
| Example 4 | ○ | ○ | A half year | Very good |
| Example 5 | ○ | ○ | Two years | Very good |
| Example 6 | ○ | ○ | Two and a half years | Good |

### Symbols indicating the results

Poor: Bubbles remained in all of the microwells and were not easily removed. Cell culture was not feasible.
Fair: Bubbles remained and some of the microwells were not feasible for carrying out cell culture even when the side surface of the vessel was lightly tapped.
Good: Bubbles remained but easily removed when the side surface of the vessel was lightly tapped, and cell culture was feasible in all of the microwells.
Very good: No single bubble remained and cell culture was feasible in all of the microwells.

Note that, in the above Examples, water contact angles were measured using DM-300, produced by Kyowa Interface Science Co., Ltd. Specifically, a 1 µL-water droplet was prepared into a microsyringe and, 1 second later from adding dropwise the droplet, a contact angle was measured by the θ/2 method from the shape of the droplet added dropwise.

### <Example 7>

A polystyrene cell culture vessel with 25 (5 x 5) microwells formed on the bottom thereof was produced by injection molding, wherein the microwell has a circular opening (R₁₀) with a diameter of 335 µm, a circular bottom surface (R'₁₀) with a diameter of 285 µm, a depth (L₁₀) of 161 µm, the bottom surface having a downward inclined plane at θ₁₁ = about 7° to the surface of the bottom of the cell culture vessel, an inverted straight conical shape having, as a central axis, a straight line passing the centroid of the bottom surface thereof (i.e., the center of the circle) and orthogonal to a plane parallel with the bottom of the cell culture vessel, and the side surface inclined at about 20° against a straight line orthogonal to the surface of the bottom of the cell culture vessel (in other words, at the cross-section on a plane passing the centroid of the bottom surface of the microwell and orthogonal to a plane parallel with the bottom of the cell culture vessel, the side surface of the microwell is inclined at θ₁₀ = about 80° to a plane parallel with the surface of the bottom of the cell culture vessel). As shown in Figure 8, on the side surface of the microwell of the above cell culture vessel, a structure configured by linear convex parts and concave parts was formed wherein a width of the convex part (X₁₁) and a width of the concave part (X₁₂) were 25 µm and a cross-section of the convex part had a square shape having a height (Y₁₀) of 10 µm on a plane passing the centroid of the bottom surface of the microwell and orthogonal to the bottom surface thereof. The surface roughness of the bottom surface of the microwell of the above cell culture vessel had the maximum height (Ry) of 0.5 µm. Note that the surface roughness of the bottom surface of the microwell was determined by extracting measured data of the bottom surface of the microwell from the three-dimensional shape data observed with a measurement pitch of 2 µm using a high-precision measuring system (KS-1100, manufactured by KEYENCE CORPORATION) and calculating the Ry value.

The obtained cell culture vessel was hydrophilized by plasma treatment, stored for half a year, and measured for a water contact angle of the vessel surface (on which the convex-concave structure was not formed) which was found to be 50°. In contrast, the water contact angle at the part on which the structure configured by linear convex parts and concave parts was formed was measured, and which was found to be 10°. In the case of the cell culture vessel stored for 2 years after the plasma treatment, the water contact angle of the vessel surface (on which the convex-concave structure was not formed) was 60°. In contrast, the water contact angle at the part on which the structure configured by linear convex parts and concave parts was formed was measured, and which was found to be 30°.

100 µL of water was added dropwise to the microwell parts of the cell culture vessel stored for half a year after the plasma treatment, but no single bubble remained and the culture was feasible. Similarly, 100 µL of water was added dropwise to the microwell parts of the cell culture vessel stored for two years after the plasma treatment, but no single bubble remained and the culture was feasible.

### <Example 8>

In the cell culture vessel of Example 7, a polystyrene cell culture vessel with the same feature as in Example 7 was produced by injection molding, except that the width of the convex part (X₂₁) and the width of the concave part (X₂₂) were changed to 50 µm and the height (Y₂₀) of the convex part was changed to 10 µm (Figure 9).

The obtained cell culture vessel was hydrophilized by plasma treatment, stored for half a year, and measured for a water contact angle of the vessel surface (on which the convex-concave structure was not formed), and which was found to be 50°. In contrast, the water contact angle at the part on which the structure configured by linear convex parts and concave parts was formed was measured, and which was found to be 15°. In the case of the cell culture vessel stored for 2 years after the plasma treatment, the water contact angle of the vessel surface (on which the convex-concave structure was not formed) was 60°. In contrast, the water contact angle at the part on which the structure configured by linear convex parts and concave parts was formed was measured, and which was found to be 35°.

100 µL of water was added dropwise to the microwell parts of the cell culture vessel stored for half a year after the plasma treatment, but no single bubble remained and the culture was feasible. Similarly, 100 µL of water was added dropwise to the microwell parts of the cell culture vessel stored for two years after the plasma treatment, but no single bubble remained and the culture was feasible.

### <Example 9>

A polystyrene cell culture vessel with 25 (5 x 5) microwells formed on the bottom thereof was produced by injection molding, wherein the microwell has a circular opening (R₃₀) with a diameter of 525 µm, a circular bottom surface (R'₃₀) with a diameter of 285 µm, a depth (L₃₀) of 239 µm, the bottom surface having a downward inclined plane at θ₃₁ = about 7° to the surface of the bottom of the cell culture vessel, an inverted straight conical shape having, as a central axis, a straight line passing the centroid of the bottom surface thereof (i.e., the center of the circle) and orthogonal to a plane parallel with the bottom of the cell culture vessel, and the side surface inclined at about 29° against a straight line orthogonal to the surface of the bottom of the cell culture vessel (in other words, at the cross-section on a plane passing the centroid of the bottom surface of the microwell and orthogonal to the bottom surface, the side surface of the microwell is inclined at θ₃₀ = about 61° to a plane parallel with the surface of the bottom of the cell culture vessel). As shown in Figure 10, on the side surface of the microwell of the above cell culture vessel, a structure configured by linear convex parts and concave parts was formed wherein a width of the convex part (X₃₁) and a width of the concave part (X₃₂) were 25 µm and the cross-section of the convex part was an arc shape having the maximum height (Y₃₀) of 10 µm and a curvature radius of 300 µm on a plane passing the centroid of the bottom surface of the microwell and orthogonal to a plane parallel with the bottom surface thereof. The surface roughness of the bottom surface of the microwell of the above cell culture vessel had a maximum height (Ry) of 0.5 µm. Note that the surface roughness of the bottom surface of the microwell was determined by extracting measured data of the bottom surface of the microwell from the three-dimensional shape data observed with a measurement pitch of 2 µm using a high-precision measuring system (KS-1100, manufactured by KEYENCE CORPORATION) and calculating the Ry value.

The obtained cell culture vessel was hydrophilized by plasma treatment, stored for half a year, and measured for a water contact angle of the vessel surface (on which the convex-concave structure was not formed), and which was found to be 50°. In contrast, the water contact angle at the part on which the structure configured by linear convex parts and concave parts was formed was measured, and which was found to be 10°. In the case of the cell culture vessel stored for 2 years after the plasma treatment, the water contact angle of the vessel surface (on which the convex-concave structure was not formed) was 60°. In contrast, the water contact angle at the part on which the structure configured by linear convex parts and concave parts was formed was measured, and which was found to be 30°.

100 µL of water was added dropwise to the microwell parts of the cell culture vessel stored for half a year after the plasma treatment, but no single bubble remained and the culture was feasible. Similarly, 100 µL of water was added dropwise to the microwell parts of the cell culture vessel stored for two years after the plasma treatment, but no single bubble remained and the culture was feasible.

The present invention encompasses the contents described in the specification and/or drawings of JP Application No. 2013-188598, which is the basis of priority of the present application.

### REFERENCE SIGNS LIST

1: Cell Culture Vessel
2: Bottom
3: Side Wall
4, 14, 24, 34: Microwell
5, 15, 25, 35: Microwell bottom surface
6, 16, 26, 36: Microwell side surface
7, 17, 27, 37: Microwell opening
8, 18, 28, 38: Convex-concave structure
9: Inner wall
r: Opening width of cell culture vessel
R, R₁₀, R₂₀, R₃₀: Opening width of microwell
R'₁₀, R'₂₀, R'₃₀: Bottom surface width of microwell
L, L₁₀, L₂₀, L₃₀: Depth of microwell
θ, θ₁₀, θ₂₀, θ₃₀: Inclination angle of microwell side surface
θ₁₁, θ₂₁, θ₃₁: Inclination angle of microwell bottom surface
X₁, X₁₁, X₂₁, X₃₁: Width of convex part
X₂, X₁₂, X₂₂, X₃₂: Width of concave part
Y, Y₁₀, Y₂₀, Y₃₀: Height of convex part
A: Culture medium
B: Oil
C: Cell

## Claims

1. A cell culture vessel comprising a bottom and a side wall, wherein
at least one microwell for containing a cell is disposed on the bottom,
the microwell has a bottom surface, a side surface, and an opening, and
a convex-concave structure is formed on the side surface of the microwell,
wherein a height of the convex part with respect to the concave part is 5 µm or more.

2. The cell culture vessel according to Claim 1, wherein the side surface of the microwell is a hydrophilic surface.

3. The cell culture vessel according to Claim 1 or 2, wherein the convex-concave structure is configured by at least two linear convex parts and concave parts.

4. The cell culture vessel according to Claim 1 or 2, wherein the convex-concave structure is configured by at least two dot projections and/or at least two dot recessions.

5. The cell culture vessel according to any one of Claims 1 to 4, wherein an opening width of the opening of the microwell is 0.1 mm or more and 1 mm or less.

6. The cell culture vessel according to any one of Claims 1 to 5, wherein the bottom surface of the microwell is a smooth surface.

7. The cell culture vessel according to Claim 6, wherein a surface roughness of the bottom surface of the microwell has a maximum height (Ry) of 3 µm or less.

8. The cell culture vessel according to any one of Claim 3 and Claims 5 to 7, wherein, in the convex-concave structure configured by at least two linear convex parts and concave parts, a width of the convex part and a width of the concave part range from 1/320 to 1/4 of a length of an outer peripheral portion of the opening.

9. The cell culture vessel according to any one of Claim 3 and Claims 5 to 8, wherein, in the convex-concave structure configured by at least two linear convex parts and concave parts, a height of the convex part with respect to the concave part ranges from 1/1000 to 1/1.5 of the opening width of the opening.

10. The cell culture vessel according to any one of Claim 3 and Claims 5 to 9, wherein, in the convex-concave structure configured by at least two linear convex parts and concave parts, a ratio of the height of the convex part to the width of the convex part ranges from 0.1 to 1.5.

11. The cell culture vessel according to any one of Claim 3 and Claims 5 to 10, wherein, in the convex-concave structure configured by at least two linear convex parts and concave parts, a width of the convex part and a width of the concave part range from 15 to 70 µm.

12. The cell culture vessel according to any one of Claim 3 and Claims 5 to 11, wherein, in the convex-concave structure configured by at least two linear convex parts and concave parts, a height of the convex part with respect to the concave part ranges from 1 to 70 µm.

13. The cell culture vessel according to any one of Claim 3 and Claims 5 to 12, wherein
the bottom surface of the microwell has an inverted conical shape having a downward inclined plane to the surface of the bottom of the cell culture vessel,
the side surface of the microwell is inclined against a straight line orthogonal to the surface of the bottom of the cell culture vessel, and
in the convex-concave structure configured by at least two linear convex parts and concave parts, a cross-section of the convex part has a square shape or an arc shape on a plane passing the centroid of the bottom surface of the microwell and orthogonal to a plane parallel with the bottom of the cell culture vessel.

14. The cell culture vessel according to any one of Claims 1 to 13, wherein
the bottom of the cell culture vessel, including the microwell and a circumference of the microwell, has a hydrophilic surface.

15. The cell culture vessel according to any one of Claims 2 to 14, wherein a water contact angle of the hydrophilic surface is 70° or less.

## Patentansprüche

1. Zellkulturgefäß, das einen Boden und eine Seitenwand umfasst, wobei
wenigstens eine Mikrovertiefung zum Enthalten einer Zelle auf dem Boden angeordnet ist,
die Mikrovertiefung eine Bodenoberfläche, eine Seitenoberfläche und eine Öffnung aufweist, und
eine konvex-konkave Struktur an der Seitenoberfläche der Mikrovertiefung ausgebildet ist,
wobei eine Höhe des konvexen Teils hinsichtlich des konkaven Teils 5 µm oder mehr beträgt.

2. Zellkulturgefäß nach Anspruch 1, wobei die Seitenoberfläche der Mikrovertiefung eine hydrophile Oberfläche ist.

3. Zellkulturgefäß nach Anspruch 1 oder 2, wobei die konvex-konkave Struktur durch wenigstens zwei lineare konvexe Teile und konkave Teile konfiguriert ist.

4. Zellkulturgefäß nach Anspruch 1 oder 2, wobei die konvex-konkave Struktur durch wenigstens zwei Punktvorsprünge und/oder wenigstens zwei Punktvertiefungen konfiguriert ist.

5. Zellkulturgefäß nach einem der Ansprüche 1 bis 4, wobei eine Öffnungsbreite der Öffnung der Mikrovertiefung 0,1 mm oder mehr und 1 mm oder weniger beträgt.

6. Zellkulturgefäß nach einem der Ansprüche 1 bis 5, wobei die Bodenoberfläche der Mikrovertiefung eine glatte Oberfläche ist.

7. Zellkulturgefäß nach Anspruch 6, wobei eine Oberflächenrauigkeit der Bodenoberfläche der Mikrovertiefung eine maximale Höhe (Ry) von 3 µm oder weniger aufweist.

8. Zellkulturgefäß nach einem der Ansprüche 3 und 5 bis 7, wobei in der konvex-konkaven Struktur, die durch wenigstens zwei lineare konvexe Teile und konkave Teile konfiguriert ist, eine Breite des konvexen Teils und eine Breite des konkaven Teils in einem Bereich von 1/320 bis 1/4 einer Länge eines äußeren Umfangsabschnitts der Öffnung liegen.

9. Zellkulturgefäß nach einem der Ansprüche 3 und 5 bis 8, wobei in der konvex-konkaven Struktur, die durch wenigstens zwei lineare konvexe Teile und konkave Teile konfiguriert ist, eine Höhe des konvexen Teils hinsichtlich des konkaven Teils in einem Bereich von 1/1000 bis 1/1,5 der Öffnungsbreite der Öffnung liegt.

10. Zellkulturgefäß nach einem der Ansprüche 3 und 5 bis 9, wobei in der konvex-konkaven Struktur, die durch wenigstens zwei lineare konvexe Teile und konkave Teile konfiguriert ist, ein Verhältnis der Höhe des konvexen Teils zu der Breite des konvexen Teils in einem Bereich von 0,1 bis 1,5 liegt.

11. Zellkulturgefäß nach einem der Ansprüche 3 und 5 bis 10, wobei in der konvex-konkaven Struktur, die durch wenigstens zwei lineare konvexe Teile und konkave Teile konfiguriert ist, eine Breite des konvexen Teils und eine Breite des konkaven Teils in einem Bereich von 15 bis 70 µm liegen.

12. Zellkulturgefäß nach einem der Ansprüche 3 und 5 bis 11, wobei in der konvex-konkaven Struktur, die durch wenigstens zwei lineare konvexe Teile und konkave Teile konfiguriert ist, eine Höhe des konvexen Teils hinsichtlich des konkaven Teils in einem Bereich von 1 bis 70 µm liegt.

13. Zellkulturgefäß nach einem der Ansprüche 3 und 5 bis 12, wobei
die Bodenoberfläche der Mikrovertiefung eine umgekehrte konische Form aufweist, die eine nach unten geneigte Ebene zu der Oberfläche des Bodens des Zellkulturgefäßes aufweist,
die Seitenoberfläche der Mikrovertiefung gegen eine gerade Linie orthogonal zu der Oberfläche des Bodens des Zellkulturgefäßes geneigt ist, und
in der konvex-konkaven Struktur, die durch wenigstens zwei lineare konvexe Teile und konkave Teile konfiguriert ist, ein Querschnitt des konvexen Teils eine Quadratform oder eine Bogenform auf einer Ebene aufweist, die durch den Schwerpunkt der Bodenoberfläche der Mikrovertiefung verläuft und orthogonal zu einer Ebene parallel zu dem Boden des Zellkulturgefäßes ist.

14. Zellkulturgefäß nach einem der Ansprüche 1 bis 13, wobei
der Boden des Zellkulturgefäßes, einschließlich der Mikrovertiefung und eines Umfangs der Mikrovertiefung, eine hydrophile Oberfläche aufweist.

15. Zellkulturgefäß nach einem der Ansprüche 2 bis 14, wobei ein Wasserberührungswinkel der hydrophilen Oberfläche 70° oder weniger beträgt.

## Revendications

1. Récipient de culture cellulaire comprenant un fond et une paroi latérale, dans lequel
au moins un micropuits destiné à contenir une cellule est disposé sur le fond,
le micropuits a une surface inférieure, une surface latérale et une ouverture, et
une structure convexe-concave est formée sur la surface latérale du micropuits,
dans lequel une hauteur de la partie convexe par rapport à la partie concave est de 5 µm ou plus.

2. Récipient de culture cellulaire selon la revendication 1, dans lequel la surface latérale du micropuits est une surface hydrophile.

3. Récipient de culture cellulaire selon la revendication 1 ou 2, dans lequel la structure convexe-concave est conçue par au moins deux parties convexes et parties concaves linéaires.

4. Récipient de culture cellulaire selon la revendication 1 ou 2, dans lequel la structure convexe-concave est conçue par au moins deux saillies de points et/ou par au moins deux renfoncements de points.

5. Récipient de culture cellulaire selon l'une quelconque des revendications 1 à 4, dans lequel une largeur d'ouverture de l'ouverture du micropuits est de 0,1 mm ou plus et de 1 mm ou moins.

6. Récipient de culture cellulaire selon l'une quelconque des revendications 1 à 5, dans lequel la surface inférieure du micropuits est une surface lisse.

7. Récipient de culture cellulaire selon la revendication 6, dans lequel une rugosité de surface de la surface inférieure du micropuits a une hauteur maximale (Ry) de 3 µm ou moins.

8. Récipient de culture cellulaire selon l'une quelconque des revendications 3 et 5 à 7, dans lequel, dans la structure convexe-concave conçue par au moins deux parties convexes et parties concaves linéaires, une largeur de la partie convexe et une largeur de la partie concave est comprise entre 1/320 et 1/4 de la longueur d'une partie périphérique extérieure de l'ouverture.

9. Récipient de culture cellulaire selon l'une quelconque des revendications 3 et 5 à 8, dans lequel, dans la structure convexe-concave conçue par au moins deux parties convexes et parties concaves linéaires, une hauteur de la partie convexe par rapport à la partie concave la pièce est comprise entre 1/1000 et 1/1,5 de la largeur d'ouverture de l'ouverture.

10. Récipient de culture cellulaire selon l'une quelconque des revendications 3 et 5 à 9, dans lequel, dans la structure convexe-concave conçue par au moins deux parties convexes et parties concaves linéaires, un rapport de la hauteur de la partie convexe à la largeur de la partie convexe est comprise entre 0,1 et 1,5.

11. Récipient de culture cellulaire selon l'une quelconque des revendications 3 et 5 à 10, dans lequel, dans la structure convexe-concave conçue par au moins deux parties convexes et parties concaves linéaires, une largeur de la partie convexe et une largeur de la partie concave est comprise entre 15 et 70 µm.

12. Récipient de culture cellulaire selon l'une quelconque des revendications 3 et 5 à 11, dans lequel, dans la structure convexe-concave conçue par au moins deux parties convexes et parties concaves linéaires, une hauteur de la partie convexe par rapport à la partie concave la partie est comprise entre 1 et 70 µm.

13. Récipient de culture cellulaire selon l'une quelconque des revendications 3 et 5 à 12, dans lequel
la surface inférieure du micropuits a une forme conique inversée ayant un plan incliné vers le bas par rapport à la surface du fond du récipient de culture cellulaire,
la surface latérale du micropuits est inclinée par rapport à une ligne droite orthogonale à la surface du fond du récipient de culture cellulaire, et
dans la structure convexe-concave conçue par au moins deux parties convexes et parties concaves linéaires, une section transversale de la partie convexe présente une forme carrée ou une forme d'arc sur un plan passant le centroïde de la surface inférieure du micropuits et orthogonale à un plan parallèle au fond du récipient de culture cellulaire.

14. Récipient de culture cellulaire selon l'une quelconque des revendications 1 à 13, dans lequel
le fond du récipient de culture cellulaire, comprenant le micropuits et une circonférence du micropuits, a une surface hydrophile.

15. Récipient de culture cellulaire selon l'une quelconque des revendications 2 à 14, dans lequel un angle de contact de l'eau de la surface hydrophile est de 70° ou moins.
